# EUROPEAN PATENT SPECIFICATION

(11) **EP 4 171 757 B1**
(45) Date of publication and mention of the grant of the patent: **02.09.2026**
(21) Application number: 21732803.8
(22) Date of filing: 25.06.2021
(51) Int. Cl.: A61Q 11/00, A61K 8/34, A61K 8/73

(54) **METHODS AND PREBIOTIC COMPOUNDS FOR IMPROVING ORAL HYGIENE AND RELATED ORAL CARE COMPOSITIONS**
VERFAHREN UND PRÄBIOTISCHE VERBINDUNGEN ZUR VERBESSERUNG DER MUNDHYGIENE UND ENTSPRECHENDE MUNDPFLEGEMITTEL
MÉTHODES ET COMPOSÉS PRÉBIOTIQUES POUR AMÉLIORER L'HYGIÈNE BUCCALE ET COMPOSITIONS D'HYGIÈNE BUCCALE CORRESPONDANTES

(30) Priority: 25.06.2020 NL 2025915
(43) Date of publication of application: 03.05.2023
(73) Proprietor: S&C Consultancy, 11-731 Sorkwity (PL)
(72) Inventor: GONRY, Patrick, 4631 SL Hoogerheide (NL); PIOTROWSKA, Anna, 4631 SL Hoogerheide (NL)
(74) Representative: De Clercq & Partners
(86) International application number: PCT/EP2021/067565
(87) International publication number: WO 2021/260202

(56) References cited:
- WO-A1-2007/072131
- WO-A1-2007/077210
- WO-A1-2015/000082
- BE-A3- 1 018 740
- JP-A- 2001 278 758
- KR-A- 20170 114 197

## Description

### Field of the invention

The present invention is situated in the field of oral hygiene and oral healthcare. In particular, the present invention is as defined in the appended claims and provides methods for improving oral health and related oral care compositions and uses thereof, wherein the oral care composition comprises a prebiotic compound for improving oral health, particularly by protecting the oral microbiota from being disrupted or imbalanced, and/or by maintaining a healthy oral microbiota in the presence of a disrupting or imbalancing factor, and/or by restoring, re-establishing or rebalancing a disrupted or imbalanced oral microbiota.

### Background of the invention

The oral cavity or mouth is colonized with a large amount of both beneficial and harmful bacteria, belonging to a wide variety of different species. A healthy and balanced oral microbiota is dominated by beneficial bacteria, which are believed to interfere with the growth and proliferation of the pathogenic oral bacteria. Some species of oral pathogenic bacteria have been implicated in tooth decay (caries) (e.g. *Streptococcus mutans*) or in the development of periodontal diseases. When the equilibrium is compromised and the oral microbiota becomes imbalanced, pathologies and disorders such as dental caries, gingivitis or periodontitis may develop. Several factors may cause a disruption of the healthy oral microbiota. For instance, food sugars may be converted by acidogenic bacteria into acids, which damages the teeth. In addition, the acidic environment may promote the growth of aciduric and acidogenic (cariogenic) bacteria. See e.g. the disclosure of WO 2007/077210 A1 (WIKSTROEM MAUDE [SE]; ALMSTAAHL ANNICA [SE]) 12 July 2007 (2007-07-12).

Many oral care products aim to eliminate the bacteria in the mouth, or even sterilize the mouth. These products not only kill the harmful bacteria, but affect the beneficial bacteria that are useful and necessary for maintaining a healthy oral cavity.

Maintaining the balance of the oral microflora is important as it can prevent the occurrence and progression of certain oral diseases. While prebiotics are generally known for influencing the composition of the gastrointestinal microflora, little attention has been directed to using a similar prebiotic strategy to encourage beneficial oral bacteria. In oral health care, the emphasis has been on avoiding and promptly removing compounds, like sucrose, that encourage harmful oral bacteria, and/or by using non-specific antibacterial agents, which eliminate both harmful and beneficial bacteria.

There is thus a need for ways to maintain, protect and/or increase the amount of the beneficial bacteria in the oral cavity, particularly concomitantly with controlling or reducing the number of harmful bacteria in the mouth or preventing their growth, and thus to prevent oral cavity disorders. Also, there is a need in the art for compounds, compositions and methods to restore and/or rebalance a disturbed oral microbiota and/or to mitigate the disruption of the oral microbiota.

### Summary of the invention

Any definitions such as "A method for treating, protecting, maintaining and/or restoring a healthy microbiota or a beneficial bacteria population in an oral cavity of a subject, comprising administering to said oral cavity a prebiotic compound..." or a related definition like e.g. "use of a prebiotic compound for treating, protecting, maintaining and/or restoring" is to be literally regarded as reference to a "prebiotic compound for use".

The inventors have surprisingly found that prebiotic compounds, such as when present in an oral care product, are able to negate or diminish the negative effect of an oral microbiota disrupting compound on an oral bacterial population, particularly on the beneficial bacterial population. Indeed, the prebiotic compound present in a mouthcare product was found to maintain and protect a balanced and healthy oral microflora from disruption by disrupting food components. In addition, the prebiotic compound present in a mouthcare product comprising an antimicrobial compound and a prebiotic compound (e.g. inulin) was found to protect and maintain the beneficial oral bacteria (e.g. *St. salivarius*) from the presence of the anti-microbial compound, thus preventing the disruption or imbalancing of the oral microbiota. In addition, a mouthcare product comprising a prebiotic compound was found to restore an imbalanced oral bacterial population to a balanced, healthy state. The prebiotic compound, such as when incorporated in an oral care product, is thus able to protect the oral bacterial population from external harm, to support the oral microbiota under stress conditions (such as in the presence of an antimicrobial compound) and to rebalance and restore the oral microbiota after it has been subjected to such stress conditions. Stated differently, the prebiotic compound, such as when incorporated in an oral care product promotes a healthy balance of microorganisms in the oral cavity, such as by maintaining, supporting, or stimulating the growth and proliferation of beneficial bacteria and/or by inhibiting the ability of pathogenic bacteria to grow in the oral cavity, thus preventing or mitigating oral cavity disorders.

The present invention thus generally relates to a prebiotic functional formula in oral hygiene applications, and related methods and compositions, wherein the prebiotic compound as envisaged herein can inhibit and prevent the growth of potential pathogenic bacteria, and promote the growth of beneficial bacteria, particularly when the oral microbiota is or has been contacted with an oral microbiota disrupting compound.

A first aspect of the disclosure provides a method for protecting, maintaining and/or restoring a healthy microbiota or a beneficial bacteria population in an oral cavity of a subject, comprising administering to or contacting said oral bacterial population with an effective amount of a prebiotic compound, particularly a composition comprising an effective amount of a prebiotic compound. In particular, the method as envisaged herein is a method for at least partially negating the effect of an oral microbiota disrupting compound on an oral bacterial population, comprising administering to or contacting the oral cavity which is or has been contacted with an oral microbiota disrupting compound, an effective amount of a prebiotic compound, particularly composition comprising an effective amount of a prebiotic compound. In particular, the method as envisaged herein is a method for preventing or inhibiting the growth of potential pathogenic, harmful or undesirable bacteria in an oral cavity of a subject, comprising administering to or contacting said oral bacterial population with an effective amount of a prebiotic compound, particularly a composition comprising an effective amount of a prebiotic compound.

The prebiotic compound is, for the purpose of defining this invention, synonymously defined as and limited to a non-digestible oligo- or polysaccharide with a degree of polymerization between 2 and 100, such as a non-digestible oligosaccharide with a degree of polymerization between 2 and 20. In particular embodiments, the prebiotic compound is selected from the group consisting of fructan, galactan, kefiran and non-digestible oligosaccharides, such as galactooligosaccharides, xylooligosaccharides, human milk oligosaccharides, or isomaltooligosaccharides , preferably wherein the prebiotic compound is inulin or fructooligosaccharides.

In particular embodiments, the method comprises administering an oral care composition to the oral cavity of a subject, wherein said composition comprises between 0.1 and 20 wt%, preferably between 0.5 and 10 wt%, of said prebiotic compound, based on the total weight of the composition. In particular embodiments, said oral care composition is a mouth wash, a tooth paste or tooth gel, a mouth spray, a powder, a lozenge, an oral tablet, or chewing gum.

In particular embodiments, the composition further comprises an oral microbiota disrupting compound, such as ethanol, a surfactant and/or an antimicrobial agent, such as triclosan, chlorhexidine or a quaternary ammonium compound.

In certain embodiments, prior to administering the oral care composition, the oral cavity comprises a disrupted or imbalanced oral microbiota, such as an oral microbiota disturbed or disrupted by ethanol, sugars or acids or by food products comprising these compounds. In particular embodiments, the method is a method for protecting, maintaining and/or restoring a healthy microbiota or a beneficial bacteria population in an oral cavity of a subject, comprising administering to the oral cavity which has previously been contacted with an oral microbiota disrupting compound, such as ethanol, an antimicrobial agent, an acid, or a sugar.

In certain embodiments, the composition comprising an effective amount of a prebiotic compound is administered at least once daily to the oral cavity of the subject, such as during each oral hygienic treatment.

Another related aspect of the present invention provides a prebiotic compound as envisaged herein for use in protecting, maintaining and/or restoring a healthy microbiota and/or a beneficial bacteria population in the oral cavity of a subject, for preventing or inhibiting the growth of potential pathogenic, harmful or undesirable bacteria in an oral cavity of a subject, or for use in preventing an oral cavity disorders. A further, related aspect of the present invention provides an oral care composition comprising an effective amount of a prebiotic compound as envisaged herein, for use in protecting, maintaining and/or restoring a healthy microbiota and/or a beneficial bacteria population in the oral cavity of a subject, for preventing or inhibiting the growth of potential pathogenic, harmful or undesirable bacteria in an oral cavity of a subject, or for use in preventing an oral cavity disorders. The prebiotic compound is particularly a non-digestible oligo- or polysaccharide with a degree of polymerization between 2 and 100, such as a non-digestible oligosaccharide with a degree of polymerization between 2 and 20. In particular embodiments, the prebiotic compound is selected from the group consisting of fructan, galactan, kefiran and non-digestible oligosaccharides, such as galactooligosaccharides, xylooligosaccharides, human milk oligosaccharides, or isomaltooligosaccharides, preferably wherein the prebiotic compound is inulin or fructooligosaccharides.

It is particularly understood that the prebiotic compound at least partially negates the effect of a microbiota disrupting compound, such as ethanol, an antimicrobial agent, a surfactant, an acid, or a sugar; said microbiota disrupting compound being in contact with said oral cavity simultaneously with or prior to administration of the oral care composition. In certain embodiments, the oral microbiota is simultaneously contacted with a microbiota disrupting compound.

Another related aspect of the present invention provides an oral care composition, comprising an effective amount of at least one prebiotic compound.

In particular embodiments, the oral care composition further comprises a microbiota disrupting compound, preferably ethanol, an antimicrobial agent, such as triclosan, chlorhexidine or a quaternary ammonium compound, or a surfactant. The prebiotic compound is particularly a non-digestible oligo- or polysaccharide with a degree of polymerization between 2 and 100, such as a non-digestible oligosaccharide with a degree of polymerization between 2 and 20. In particular embodiments, the prebiotic compound is selected from the group consisting of fructan, galactan, kefiran and non-digestible oligosaccharides, such as galactooligosaccharides, xylooligosaccharides, human milk oligosaccharides, or isomaltooligosaccharides, preferably wherein the prebiotic compound is inulin or fructooligosaccharides.

In particular embodiments, the oral care composition comprises between 0.1 and 20 wt%, preferably between 0.5 and 10 wt%, of the prebiotic compound, based on the weight of the composition.

The oral care composition as envisaged herein may further comprise one or more additional ingredients, such as a fluoride ion source, abrasives, diluents, pH modifying or buffering agents, surfactants, foaming agents, thickening agents, humectants, sweeteners, pigments, flavouring agents, fragrances, preserving agents.

The oral care composition as envisaged herein may preferably be a mouth wash, a tooth paste or tooth gel, a mouth spray, a powder, a lozenge, an oral tablet, or chewing gum.

In certain embodiments, the oral care composition is a mouthwash comprising an effective amount of the prebiotic compound as envisaged herein and further comprising an aqueous ethanol solution, a surfactant or an antimicrobial agent.

In certain embodiments, the oral care composition is a toothpaste comprising an effective amount of the prebiotic compound as envisaged herein and further comprising an antimicrobial agent or a surfactant.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

In the present application, the percentages are given by weight, unless otherwise stated.

The inventors have surprisingly found that prebiotic compounds can be used to maintain the oral microbiome in a healthy, balanced state and/or to return the oral microbiome to a healthy, balanced state, wherein the prebiotic compounds provide the beneficial bacteria with sufficient nutrients to outcompete the undesirable bacteria and/or to increase more quickly in number. Within this context, the present invention generally relates to the use of a prebiotic functional formula in oral hygiene and oral care applications, and related methods and compositions, wherein the prebiotic compound as envisaged herein can promote the growth of beneficial bacteria and inhibit or prevent the growth of potential pathogenic and undesirable bacteria, particularly when the oral microbiota is or has been contacted with an oral microbiota disrupting compound. The present application provides methods and compositions for stabilizing or restabilizing an oral bacterial population, which is or has been in contact with an oral microbiota disrupting compound, wherein at least one prebiotic compound negates or diminishes the negative effect of said oral microbiota disrupting compound on said oral microbiota so that said oral microbiota does not further lead to an oral cavity disorder, but is restored or maintained in a healthy, balanced state. The inventors have found that the addition of a prebiotic compound in an oral care composition has several beneficial effects on the oral bacterial population, particularly on the beneficial bacteria which dominate a healthy oral microbiota. The prebiotic compound was found to protect a healthy oral bacterial population from external harm or stress conditions, such as when contacted with sugar rich foods, or with an antimicrobial agent or disinfectant, such as ethanol, and to support a healthy oral bacterial population under such stress conditions. In addition, the oral bacterial population is restored and rebalanced after it has been subjected to external harm or stress conditions. It is thus understood that the different embodiments of the methods and compositions envisaged in the present application promote a healthy balance of microorganisms in the oral cavity, by encouraging the growth of beneficial bacteria and inhibiting or preventing the ability of pathogenic and opportunistic microorganisms to grow or to invade the oral cavity. The different embodiments of the methods and compositions envisaged in the present application thus provide health benefits of promoting, restoring, reinforcing and maintaining the defence mechanisms provided by a balanced and healthy microbiota, thus preventing or mitigating oral cavity disorders. The term "to prevent" is generally understood to mean the fact of reducing the risk of onset of a phenomenon, in particular an oral cavity disorder. While conventional prebiotics are ingestible ingredients that selectively support, upon ingestion, the growth of beneficial gut or colon bacteria, it has surprisingly been found that the compounds envisaged herein exhibit prebiotic activity on the beneficial bacteria in the oral cavity, i.e. a completely different microbiome from that of the gut or colon, without requiring ingestion and despite the short contact time typical for oral hygiene applications.

As used herein, the term "oral care composition" refers to a composition adapted for oral hygiene purposes, i.e. for cleaning or sanitizing the oral cavity of a subject. The term "oral cavity" as used herein includes not only the mouth or cavity itself, but also includes the teeth, gingiva, periodontal pockets, cheeks, tongue, mucosa, tonsils, any implants, and any device or structure which is placed into the oral cavity. Oral care compositions particularly aim to prevent and/or treat oral cavity related conditions, disorders or diseases, such as dental caries (cavities), halitosis, gingivitis, mouth ulcers including aphthous stomatitis (canker sores), candidiasis and periodontal diseases. An oral care composition as used herein designate a product which, when used, is typically retained in the oral cavity for a time sufficient to contact substantially all of the oral cavity surfaces, but which is not intentionally ingested.

As used herein, the term "subject" typically refers to a mammal, preferably a human. The subject may also be a domesticated mammal such as a cat, dog or horse.

As used herein, the term "healthy oral microbiota" refers to a health-associated oral microbial population, i.e. the microbial population of an oral cavity that is in a healthy state, free of any oral cavity diseases or disorders. A healthy oral microbiota is typically dominated by beneficial bacteria. In addition, beneficial bacteria in the oral cavity may have beneficial effects on the oral health themselves. In an imbalanced or disrupted oral microbiota, harmful bacteria or bacteria related to oral cavity disorders occur in greater abundance than in a healthy oral microbiota. The state or condition of an oral microbiota may be determined by assessing the bacterial species and their relative abundance in the oral microbiome, such as based on the bacteria 16s rRNA gene, which may be analysed by genomic DNA isolation, bacteria 16s rRNA gene amplification and sequencing.

The oral microbiota, particularly the healthy microbiota, may be affected, disturbed or disrupted by a so-called "microbiota disrupting compound". Such factors include compounds or means that reduce the total number of bacteria, such as antimicrobial agents or disinfectants. Other microbiota disrupting compounds or means may lead to an imbalanced oral microbiota, wherein the balance between beneficial and harmful oral bacteria is shifted towards the harmful bacteria. For instance, sugar and acids, typically occurring in food products, may promote the growth of certain harmful oral bacteria which consume the sugar thereby creating an acidic environment. Other microbiota disrupting compounds include surfactants.

A first aspect of the present application generally provides a method for protecting, maintaining and/or restoring a healthy microbiota or a beneficial bacteria population in an oral cavity of a subject, comprising administering to the oral cavity an effective amount of a prebiotic compound as envisaged herein, in particular a composition comprising an effective amount of a prebiotic compound as envisaged herein. Said method may be a method for preventing or inhibiting the growth of potential pathogenic, harmful or undesirable bacteria in an oral cavity of a subject, comprising administering to or contacting said oral bacterial population with an effective amount of a prebiotic compound, particularly a composition comprising an effective amount of a prebiotic compound. Said method may also be a method for negating or diminishing the effect of an oral microbiota disrupting compound on an oral bacterial population, comprising contacting said oral bacterial population with an effective amount of a prebiotic compound, in particular with a composition comprising an effective amount of a prebiotic compound. Stated differently, the present invention relates to a method for protecting, maintaining and/or restoring a healthy microbiota or a beneficial bacteria population in an oral cavity of a subject, comprising administering to the oral cavity, which is or has been contacted with an oral microbiota disrupting compound an effective amount of a prebiotic compound, in particular a composition comprising an effective amount of a prebiotic compound.

As used herein, the term "effective amount" is generally understood as an amount of the specified component sufficient to have the specified properties under the specified conditions. The term "an effective amount of a prebiotic compound", also referred to as a prebiotically effective amount or an amount effective to exhibit prebiotic activity, thus relates to an amount of a prebiotic compound sufficient to increase the bacterial count of one or more beneficial microorganisms or to decrease the bacterial count of one or more pathogenic microorganisms, such as by at least 10%, at least 20% or more. An effective amount of a prebiotic compound, or a prebiotically effective amount, may also relate to an amount of a prebiotic compound sufficient to decrease the disturbance of the total oral microbiome, such as by at least 20%, at least 30%, or more, in particular by evaluating the percentage of species of the total microbiome wherein the abundance has changed more than 30% upon contact with a microbiota disrupting compound. This may be determined by plate count methods, or by assessing the bacterial species and their relative abundance in the oral cavity, such as based on the bacteria 16s rRNA gene, which may be analysed by genomic DNA isolation, bacteria 16s rRNA gene amplification and sequencing, in the presence or absence of the prebiotic compound, particularly following contacting the oral cavity with a microbiota disturbing compound.

A prebiotic compound as envisaged herein is an organic substance that is not digested by the host or subject, but is able to selectively promote the growth and reproduction of one or more beneficial bacteria, particularly of one or more beneficial bacteria in the oral cavity. In the context of the present invention, prebiotic compounds are non-digestible oligo- or polysaccharides or sugar alcohols. They typically are water-soluble compounds and, particularly, do not form gels in water. More in particular, a prebiotic compound as envisaged herein is a non-digestible oligosaccharide or polysaccharide consisting of between 2 and 100 monosaccharide units, preferably between 2 and 60 monosaccharide units, or stated differently, having a degree of polymerization, or DP, between 2 and 100, preferably between 2 and 60. Even more preferred prebiotic compounds are non-digestible oligosaccharides comprising between 2 and 20 monosaccharide units, preferably between 2 and 10 monosaccharide units, i.e. having a DP of 2 to 20, preferably a DP of 2 to 10. Advantageously, smaller oligosaccharides are more effective in exhibiting a prebiotic effect given the typical short contact times in oral hygiene applications.

In particular embodiments of the present invention, the prebiotic compound is selected from the group consisting of fructan, galactan, and non-digestible oligosaccharides, such as galactooligosaccharides, xylooligosaccharides, human milk oligosaccharides, isomaltooligosaccharides and kefiran.

Preferably, the prebiotic compound is a fructan, galactan or a non-digestible oligosaccharide. Preferably, the prebiotic compound belongs to the fructans or fructooligosaccharides, i.e. a compound made up of repetitive fructose moieties. A particularly preferred fructan is inulin, a fructose oligo- or polymer made up of beta-(2,1) linked fructose moieties and a chain terminating glucose moiety. Galactooligosaccharides are galactose-containing oligosaccharides commonly produced from lactose using the transgalactosylase activity of the enzyme β-galactosidase, typically comprising a terminal glucose unit.

Particularly, the composition as envisaged herein comprises the prebiotic compound in an amount effective to protect, maintain and/or restore a healthy oral microbiota and/or in an amount to promote the growth of beneficial endogenous bacteria in the oral cavity and/or in an amount to prevent or to inhibit the growth of harmful or potentially pathogenic bacteria in the oral cavity. In particular embodiments, the composition as envisaged herein comprises between 0.1 and 20 wt%, preferably between 0.5 and 10 wt% or between 1 and 5 wt%, of said prebiotic compound, based on the weight of the composition.

It is understood that, in various embodiments, the prebiotic compound, particularly in an amount effective to exhibit prebiotic activity or in a prebiotic effective amount, is capable of being selectively utilized by a microorganism in the oral cavity to provide a health benefit, in particular by the selective stimulation of beneficial microorganisms in the oral cavity, competitively against and to the detriment of the growth of undesirable, or pathogenic microorganisms, thus preventing and/or mitigating oral cavity disorders. Such a health benefit can particularly include one or more of (i) helping beneficial microorganisms to flourish in the oral cavity, (ii) protecting and promoting the balance and diversity of a healthy microbiota in the oral cavity, and/or (iii) increasing the defences against excessive growth or infection by harmful bacteria and/or increasing the resistance against oral cavity disorders.

It is further understood that the health benefit provided by the prebiotic compound does not require administering, particularly simultaneously, an effective amount of beneficial bacteria to the oral cavity. In particular embodiments, the oral care composition comprising an effective amount of a prebiotic compound as envisaged herein, does not comprise an effective amount of beneficial or probiotic bacteria.

An oral microbiota disrupting compound as envisaged herein may be any compound which disturbs or disrupts an oral bacterial population by reducing the total bacterial count or by disrupting the balance between beneficial and harmful bacteria of a healthy oral bacterial population to a less healthy state, favouring the harmful bacteria. Said oral microbiota disrupting compound may be ethanol, an antimicrobial agent, a surfactant, an acid, or a sugar.

In particular embodiments, said prebiotic compound is administered together with said oral microbiota disrupting compound, such as when a composition comprises said prebiotic compound and an oral microbiota disrupting compound, such as ethanol, an antimicrobial agent or a surfactant. The provision of oral microbiota disrupting compounds in a composition for oral administration can be justified, for instances by other purposes such as oral hygiene purposes. Advantageously, the prebiotic compound protects the oral bacterial population from the microbiota disrupting compound, and mitigates or negates the latter compound's negative effect. In particular embodiments, said prebiotic compound is administered to an oral cavity which has been contacted with an oral microbiota disrupting compound, such as an acid or a sugar, for instance an acid- or sugar-rich food product, thus leading to an imbalanced or disrupted oral bacterial population, prior to the administration of the composition comprising the prebiotic compound. Advantageously, the prebiotic compound contributes to restoring the disrupted and imbalanced oral population to a healthy state.

As envisaged herein, the oral bacterial population may thus be contacted with an oral microbiota disrupting compound prior to or simultaneously with the administration of an effective amount of the prebiotic compound. Without wishing to be bound by theory, the prebiotic compound may promote the growth of the beneficial oral bacteria while not simultaneously promoting the growth of the harmful oral bacteria, thereby restoring the disrupted oral bacterial population to a healthy and balanced state.

In particular, the methods as envisaged herein comprises contacting an oral cavity or an oral bacterial population with an oral care composition according to another aspect of the present invention, as further described herein.

In certain embodiments of the methods envisaged herein, the composition comprising an effective amount of a prebiotic compound, in particular the oral care composition as envisaged herein, is administered at least once daily to the oral cavity of the subject. For instance, the composition comprising an effect amount of a prebiotic compound may be used by a subject in their daily oral care or oral hygiene regimen. The composition comprising an effective amount of a prebiotic compound, in particular the oral care composition as envisaged herein may be administered by applying the composition to the oral cavity using a brush, by rinsing the oral cavity with the composition in the form of a mouthwash, by spraying the composition into the oral cavity using, for example, an atomizer, or by any other suitable dosage or application form, such as by contacting the oral cavity with a lozenge, oral tablet or a chewing gum. Typically, administering of the prebiotic compound, such as contained in the oral care composition envisaged herein, does not comprise ingestion of the prebiotic compound. In particular embodiments, administering of the prebiotic compound, such as contained in the oral care composition envisaged herein, comprises contacting the prebiotic compound with the oral cavity, particularly with the bacterial population in the oral cavity, for a period of time less than 20min, 15 min or 10 min, preferably between 20 s and 5 min, such as between 30 s and 4 min.

Another related aspect of the present application provides for an oral care composition comprising an effective amount of at least one prebiotic compound as envisaged herein.

In particular embodiments, the prebiotic compound is selected from the group consisting of fructan, galactan, and non-digestible oligosaccharides, such as galactooligosaccharides, xylooligosaccharides, human milk oligosaccharides, isomaltooligosaccharides and kefiran. Preferably, the prebiotic compound is a fructan, galactan or a non-digestible oligosaccharide selected from the group of galactooligosaccharides, xylooligosaccharides, human milk oligosaccharides, and isomaltooligosaccharides. Preferably, the prebiotic compound belongs to the fructans or fructooligosaccharides, more preferably, the prebiotic compound is inulin.

In particular embodiments, the oral care composition as envisaged herein comprises the prebiotic compound in an amount effective to protect, maintain and/or restore a healthy oral microbiota and/or in an amount to promote the growth of beneficial endogenous bacteria in the oral cavity. In particular embodiments, the oral care composition as envisaged herein comprises between 0.1 and 20 wt%, preferably between 0.5 and 10 wt% or between 1 and 5 wt%, of said prebiotic compound, based on the weight of the composition.

In particular embodiments, the oral care composition as envisaged herein further comprises an oral microbiota disrupting compound. Preferably, the microbiota disrupting compound is ethanol, a surfactant, an antimicrobial agent, such as, triclosan, chlorhexidine or a quaternary ammonium compound, such as benzalkonium chloride or cetylpyridinium chloride.

Advantageously, it has been found that the combination of an antimicrobial agent (e.g. ethanol) and a prebiotic compound protects and maintains the beneficial bacterial population, thereby mitigating the effect of the antimicrobial agent on a healthy and balanced microbiota. Without wishing to be bound by theory, the prebiotic compound may promote the growth and survival of the beneficial oral bacteria while not simultaneously promoting the growth and survival of the harmful oral bacteria.

In particular embodiments, the oral care composition is a mouth wash, a tooth paste or tooth gel, a mouth spray, a powder, a lozenge, an oral tablet, or a chewing gum.

In particular embodiments, the oral care composition of the present invention may further comprise one or more orally acceptable additional ingredients. Carriers, diluents and excipients for oral compositions are generally known the art. It is understood that, in general, the skilled person is able to optimize the formulation of the compositions according to the present invention and the processes for preparing them on the basis of his general technical knowledge and taking into account the envisaged use and desired characteristics of the composition.

Suitable additional ingredients may be selected from a fluoride ion source, remineralization agents, abrasives, diluents, pH modifying or buffering agents, surfactants, foaming agents, thickening agents, humectants, sweeteners, pigments, flavouring agents, fragrances, coloring agents, and preserving agents.

Remineralization agents include calcium and phosphate containing compounds, such as tricalcium phosphate, nanoparticles of amorphous calcium phosphate (NACP), hydroxyapitite, calcium glycerophosphate and the like.

Suitable fluoride ion sources include stannous fluoride, sodium fluoride, amine fluorides and sodium monofluorophosphate. Fluoride contributes to strengthening the tooth enamel.

Suitable surfactants include sodium lauryl sulfate, sodium lauryl glutamate, sodium coconut monoglyceride sulfonate, sodium lauryl sarcosinate, sodium lauryl isoethionate, sodium laureth carboxylate and sodium dodecyl benzenesulfonate. Betaines, such as cocoamidopropyl betaine may also be used. Surfactants may contribute to an uniform distribution of the oral care composition, improving its cleansing power. They usually have foaming properties as well. Suitable foaming agents or foaming modulators include high molecular weights polyethylene glycols.

Suitable abrasives include silica, aluminium oxide, aluminium silicate, calcium carbonate or calcium phosphate. Abrasives are particularly present in toothpaste and help in cleaning the teeth, such as by promoting the removal of dental plaque and tartar, while mouthwash compositions are typically free of abrasives.

Suitable sweeteners include low cariogenic sugar substitutes, such as sugar alcohols, stevia, aspartame, saccharin and the like, and enhance the taste of the composition.

Flavoring agents and fragrances enhance the taste and/or aroma of the oral care composition. Any orally acceptable natural or synthetic flavouring agent or fragrance can be used, including but not limited to oils and essences from peppermint, eucalyptus, citrus and the like.

Suitable humectants include glycerine, sorbitol and low molecular weight polyethylene glycols. Many humectants also function as sweeteners.

Suitable pH modifying agents for maintaining the composition in an orally acceptable pH range include phosphoric and sulfonic acids and salts thereof, alkali metal hydroxides such as sodium hydroxide, carbonates such as sodium carbonate, and bicarbonates.

Suitable thickening agents include carrageenans, natural gums, such as xanthan or gum Arabic, carbomers, and cellulose polymers, such as carboxymethyl cellulose and salts thereof. They typically contribute to the desired consistency and mouth feel of the oral care composition.

In certain embodiments, the oral care composition is a mouthwash composition comprising a prebiotic compound, and, preferably, further comprising a microbiota disrupting compound, including but not limited to ethanol and/or an antimicrobial agent, and/or a surfactant. More in particular, the oral care composition is a mouthwash composition comprising a prebiotic compound as envisaged herein, particularly a prebiotic compound, selected from the group consisting of fructan, galactan, and prebiotic or non-digestible oligosaccharides, such as xylooligosaccharides, human milk oligosaccharides, isomaltooligosaccharides, kefiran or galactooligosaccharides, preferably fructan, galactan or non-digestible oligosaccharides with DP between 2 and 20 or between 2 and 10, more preferably fructan, such as inulin, particularly in a concentration of between 0.1 and 20 wt%, such as between 0.5 and 10 wt%, and further comprising an aqueous ethanol solution, a surfactant, and/or an antimicrobial agent.

In certain embodiments, the oral composition is a toothpaste, comprising a prebiotic and, preferably, further comprising a microbiota disrupting compound, including but not limited to ethanol, a surfactant and/or an antimicrobial agent. More in particular, the oral care composition is a toothpaste composition comprising a prebiotic compound as envisaged herein, particularly a prebiotic compound, selected from the group consisting of fructan, galactan, and prebiotic or non-digestible oligosaccharides, such as xylooligosaccharides, human milk oligosaccharides, isomaltooligosaccharides, kefiran, or galactooligosaccharides, preferably fructan, galactan or non-digestible oligosaccharides with DP between 2 and 20 or between 2 and 10, more preferably fructan, such as inulin, particularly in a concentration of between 0.1 and 20 wt%, such as between 0.5 and 10 wt%, and further comprising an antimicrobial agent. Preferably, the toothpaste further comprises one or more of an abrasive, a fluoride source, a surfactant, humectant, thickener and a flavouring agent.

In certain embodiments, the oral composition is a chewing gum, comprising a prebiotic compound as envisaged herein. In particular embodiments, said prebiotic compound is a fructan, galactan or non-digestible oligosaccharides with DP between 2 and 20 or between 2 and 10, more preferably fructan, such as inulin, particularly in a concentration of between 0.1 and 20 wt%, such as between 0.5 and 10 wt%. Preferably, the chewing gum further comprises one or more of conventional chewing gum polymers, a filler, a coloring agent, a flavoring agent, a sweetener, a softener, an emulsifier, an acidulant, an antioxidant, ...

Another aspect of the present invention relates to an oral care composition comprising a prebiotic compound according to the present invention for use in protecting, maintaining, restoring or re-establishing a healthy bacterial population and/or a beneficial bacteria population in the oral cavity of a subject. In certain embodiments of said use, the oral microbiota in need of protecting, maintaining, restoring or re-establishing is or has been contacted with a microbiota disrupting or disturbing compound, such as ethanol, an antimicrobial agent, a surfactant, an acid or a sugar. In certain embodiments of said use, the oral bacterial population has been imbalanced or disrupted prior to the administration of the oral care composition of the present invention, such as by sugar-containing or acidic food products, or by ethanol or another antimicrobial agent.

Another aspect of the present invention relates to an oral care composition comprising a prebiotic compound according to the present invention for use in in preventing, mitigating or treating an oral cavity disorder, such as dental caries, halitosis (malodor), gingivitis, mouth ulcers, candidiasis and periodontal diseases (such as periodontitis). In certain embodiments of said use, the oral cavity is or has been contacted with a microbiota disrupting compound, such as ethanol, an antimicrobial agent, a surfactant, an acid or a sugar. More particularly, the invention is of interest for the prevention of an oral cavity disorder in a subject. In particular embodiments, the subject is a subject susceptible to disruption of the oral cavity microbiome balance. In particular embodiments, the use is prophylactic in that the subject does not yet have an imbalance of the oral cavity bacterial population. In particular embodiments, the subject does have an oral cavity imbalance but does not suffer from an oral cavity disorder. Methods for determining oral cavity imbalance of the bacterial population are known in the art. In particular embodiments, the method comprise determining whether the subject has an oral cavity imbalance or is susceptible to an oral cavity imbalance in the bacterial population.

The present application also provides aspects and embodiments as set forth below.

In particular embodiments, the invention provides a method for at partially negating the effect of an oral microbiota disrupting compound on an oral bacterial population, comprising contacting said oral bacterial population with a composition comprising an effective amount of prebiotic compound. In particular embodiments the method is a method for protecting, maintaining and/or restoring a healthy microbiota or a beneficial bacteria population in an oral cavity of a subject, comprising administering to said oral cavity which is or has been contacted with an oral microbiota disrupting compound, a composition comprising an effective amount of a prebiotic compound. In particular embodiments of these methods, the prebiotic compound is selected from the group consisting of fructan, galactan, and non-digestible oligosaccharides, such as galactooligosaccharides, xylooligosaccharides, human milk oligosaccharides, isomaltooligosaccharides and kefiran, preferably wherein the prebiotic compound is inulin or fructooligosaccharides. In particular embodiments of these methods, the composition further comprises said oral microbiota disrupting compound. For instance, said oral microbiota disrupting compound can be ethanol, an antimicrobial agent, such as triclosan, chlorhexidine or a quaternary ammonium compound, or a surfactant.

In particular embodiments of said method, said composition comprises between 0.1 and 20 wt%, preferably between 0.5 and 10 wt%, of said prebiotic compound, based on the total weight of the composition. In particular embodiments, said composition is administered at least once daily to the oral cavity of the subject. In particular embodiments, said oral cavity has been contacted with an oral microbiota disrupting compound and said oral microbiota disrupting compound is selected from ethanol, a surfactant, an antimicrobial agent, an acid, or a sugar.

The invention also provides oral care compositions, comprising an effective amount of at least one prebiotic compound. In particular embodiments said composition further comprises a microbiota disrupting compound, preferably ethanol, an antimicrobial agent, such as triclosan, chlorhexidine or a quaternary ammonium compound, or a surfactant. Optionally, the prebiotic compound is an oligo- or polysaccharide selected from the group consisting of fructan, galactan, and non-digestible oligosaccharides, such as xylooligosaccharides, human milk oligosaccharides, isomaltooligosaccharides, galactooligosaccharides or kefiran. Further, the prebiotic compound may be inulin or fructooligosaccharides.

In particular embodiments, the composition is a composition comprising between 0.1 and 20 wt%, preferably between 0.5 and 10 wt%, of prebiotic compound, based on the weight of the composition and wherein in the composition optionally comprises one or more additional ingredients selected from a fluoride ion source, remineralization agent, abrasives, diluents, pH modifying or buffering agents, surfactants, foaming agents, thickening agents, humectants, sweeteners, pigments, flavouring agents, fragrances, preserving agents.

In particular embodiments, the composition is a mouth wash, a tooth paste or tooth gel, a mouth spray, a powder, a lozenge, an oral tablet, or chewing gum. For instance, the composition can be a mouthwash comprising a prebiotic compound, preferably selected from the group consisting of fructan, galactan, and non-digestible oligosaccharides, such as galactooligosaccharides, xylooligosaccharides, human milk oligosaccharides, isomaltooligosaccharides and kefiran, more preferably inulin, and further comprising an aqueous ethanol solution or an antimicrobial agent.

In particular embodiments, the composition is a toothpaste comprising a prebiotic compound, preferably selected from the group consisting of fructan, galactan, and non-digestible oligosacchirdes, such as galactooligosaccharides, xylooligosaccharides, human milk oligosaccharides, isomaltooligosaccharides and kefiran, more preferably inulin, and further comprising an antimicrobial agent.

The application also envisages oral care compositions as described herein, for use in protecting, maintaining and/or restoring a healthy microbiota and/or a beneficial bacteria population in the oral cavity of a subject, wherein the prebiotic compound at least partially negates the effect of a microbiota disrupting compound, such as ethanol, an antimicrobial agent, a surfactant, an acid, or a sugar; said microbiota disrupting compound being in contact with said oral cavity simultaneously with or prior to administration of the oral care composition.

The application also envisages oral care compositions as described herein, for use in preventing or mitigating an oral cavity disorder, such as caries, gingivitis, or periodontitis.

### Examples

### Example 1 - Oral microbiome disturbed with ethanol

### Experimental setup

The rebalancing/supporting effect of inulin on an oral microbiome disturbed with ethanol was evaluated by comparing the initial oral microbiome with the oral microbiome after disturbance with ethanol, as influenced by a blank mouth wash and by a mouth wash containing 2% inulin. Ethanol has an antimicrobial effect.

The blank mouth wash contained water (90%) and ethanol (10%); the prebiotic containing mouth wash contained water (88%), ethanol (10%) and inulin (2%).

The test was performed on 3 subjects. Each volunteer did not drink (except water) nor eat for at least 3 hours prior to the test. The volunteers also did not eat during the test. The blank mouth wash was tested on the first day of the test. The mouth wash containing 2% inulin was tested the next day.

DNA was collected with Zymo Research R1104/R1106 swabs with tubes. The swabs were applied for 60 seconds over the teeth, gum and tongue.

The subjects were informed about the importance and the meaning of the study. Written informed consent was obtained from all subjects prior to entry into the trial. Following criteria were used for subject selection: >= 18 years old and clinically healthy (no mouth diseases); For exclusion: oral diseases or pregnancy.

The conditions of the test were kept hygienic by sterilization of laboratory tools and glass and by boiling the water used for the mouthwash for 5 min prior to its use. Furthermore, a HEPA filter was used to limit the presence of foreign microorganisms in the air and the researcher wore gloves and mouth mask at all times.

The procedure was as follows:
1) The oral microbiome of each subject was sampled with a swab. The swab was rubbed well on gums, teeth and tong. The swab was then closed in a tube with buffer and placed immediately in a freezer at -18°C.
2) Immediately after the sampling, each subject rinsed its mouth with 30 ml of mouthwash during 30 seconds.
3) After 1 hour, the oral microbiome of each subject was sampled again with a swab. The swab was rubbed well on gums, teeth and tong. The swab was then closed in a tube with buffer and placed immediately in a freezer at -18°C.

The same procedure was repeated the next day for the mouth wash containing 2% of the prebiotic compound.

The swabs were analysed by genomic DNA isolation and bacteria 16s rRNA gene (V3-V4) PCR amplification and Illumina sequencing (Illumina MiSeq 2x300 bp).

The raw data includes Illumina sequenced reads presented as an abundance summary for all taxonomic paths and classification per unique pseudoread.

### Results

The influence on the abundance of *Streptococcus salivarius* when using mouthwash containing 10% ethanol against the same mouthwash enriched with 2% Inulin is presented in Table 1.

**Table 1. Results for stabilizing effect on Staphylococcus salivarius after using mouthwash**

| Disturbance of St salivarius after using blank mouthwash | Disturbance of St salivarius after using mouthwash with 2% prebiotic |
|---|---|
| -45,97% | -1.22% |

The disturbance on particular species was calculated by taking into account the number of species which were out of balance (i.e. wherein the abundance has changed more than 30%) and is shown in Table 2.

**Table 2. Microbiome Disturbance**

| Amount of microbiome species disturbed after using blank mouth wash | Amount of microbiome species disturbed after using mouth wash with 2% prebiotic |
|---|---|
| 75% | 57% |

A mouthwash containing 10% ethanol significantly disturbs the population of *St salivarius,* which belongs to a healthy and protective oral bacterial microbiome: as shown in Table 1 the population was decreased by almost 50%.

The same mouthwash enriched with 2% inulin maintains the abundance of *St salivarius.* The prebiotic inulin thus protects the population of *St salivarius* in the presence of ethanol.

Similarly, inulin reduces the negative impact of ethanol on the total oral microbiome, as shown in Table 2. Without inulin, ethanol disturbs 75% of the oral microbiome, while in the presence of inulin, ethanol disturbs 57% of the species.

These results demonstrate that the presence of a prebiotic compound, such as inulin, in a mouth wash containing ethanol makes the mouthwash milder and more supportive for the oral microbiome.

### Example 2 - Oral microbiome disturbed with glucose and ethanol

### Experimental setup

The rebalancing/supporting effect of inulin on an oral microbiome disturbed with glucose and ethanol was evaluated by comparing the initial oral microbiome, which has been disturbed with sugar, with the oral microbiome after further disturbance with ethanol, as influenced by a blank mouth wash and by a mouth wash containing 2% inulin. Sugar promotes the growth of micro-organisms associated with caries.

The blank mouth wash contained water (90%) and ethanol (10%); the prebiotic containing mouth wash contained water (88%), ethanol (10%) and inulin (2%).

The test was performed on 3 subjects. Each volunteer did not drink (except water) nor eat for at least 3 hours prior to the test. The volunteers also did not eat during the test. The blank mouth wash was tested on the first day of the test. The mouth wash containing 2% inulin was tested the next day.

DNA was collected with Zymo Research R1104/R1106 swabs with tubes. The swabs were applied for 60 seconds over the teeth, gum and tongue.

The subjects were informed about the importance and the meaning of the study. Written informed consent was obtained from all subjects prior to entry into the trial. Following criteria were used for subject selection: >= 18 years old and clinically healthy (no mouth diseases); For exclusion: oral diseases or pregnancy.

The conditions of the test were kept hygienic by sterilization of laboratory tools and glass and by boiling the water used for the mouthwash for 5 min prior to its use. Furthermore, a HEPA filter was used to limit the presence of foreign microorganisms in the air and the researcher wore gloves and mouth mask at all times.

The procedure was as follows:
1) The oral microbiome of each subject was sampled with a swab. The swab was rubbed well on gums, teeth and tong. The swab was then closed in a tube with buffer and placed immediately in a freezer at -18°C.
2) Immediately after the sampling each subject had to rinse their mouth with 30 ml of 50% glucose solution for 30 seconds.
3) After 1 hour, the mouth of each volunteer was rinsed with 30 ml of mouthwash with ethanol (Blank) for 30 seconds.
4) Another sample was taken after 2 hours(1h after the mouth wash treatment). The swab was rubbed well on gums, teeth and tong. The swab was then closed in a tube with buffer and placed immediately in a freezer at -18°C.

The same procedure was repeated the next day for the mouth wash containing 2% prebiotic.

The swabs were analysed by genomic DNA isolation and bacteria 16s rRNA gene (V3-V4) PCR amplification and Illumina sequencing (Illumina MiSeq 2x300 bp).

The raw data includes Illumina sequenced reads presented as an abundance summary for all taxonomic paths and classification per unique pseudoread.

### Results

The disturbance of the total microbiome was evaluated by taking into account the percentage of species of the total microbiome that are out of balance (i.e. wherein the abundance has changed more than 30%) and is shown in Table 3.

**Table 3. Microbiome disturbance after glucose and mouth wash treatmentl**

| Disturbed microbiome for blank mouth wash | Disturbed microbiome for mouth wash with inulin |
|---|---|
| 90.78% | 43.66% |

The influence on the abundance of pathogens causing dental caries is shown in Table 4. The following bacteria species were evaluated: *Streptococcus sanguinis, Streptococcus mutans, Bifidobacterium, Lactobacillus, Propionibacterium, Scardovia wiggsiae.*

**Table 4. Influence on abundance of pathogens causing dental carries**

| Blank | | 2% inulin | |
|---|---|---|---|
| 0 min | 120 min | 0 min | 120 min |
| 2.26% | 2.62% | 1.7% | 1.53% |

When the oral microbiome is disturbed with glucose and a mouth wash containing ethanol, 2 hours later about 90% of the bacteria is still out of balance. For the same test when using mouthwash containing 2% of prebiotic only about 40-45% of the total microbiome is out of balance (Table 3).

Comparison of abundance of the pathogens causing the most common oral disease (dental carries) shows that the presence of inulin tends to control the abundance of these pathogenic bacteria and can thus be seen as contributing to the prevention of caries or another oral cavity disorder.

### Example 3 - stabilizing the oral microbiome with a chewing gum comprising a prebiotic compound

### Experimental setup

The present experiment studied the restoring/rebalancing power of a prebiotic for the oral microbiota after using foodstuff/drinks associated with caries, gingivitis and other oral diseases. In order to evaluate the rebalancing/supporting effect of inulin on a disturbed oral microbiome, the oral microbiome was first disturbed with sugar, which promotes the growth of micro-organisms associated with caries, periodontitis, gingivitis, or malodor. Next, the initial disturbed oral microbiome was with the oral microbiome as influenced by a blank chewing gum, containing no prebiotic, and a chewing gum containing 2% inulin as the prebiotic compound.

The test was performed on 3 subjects. Each volunteer did not drink (except water) or eat for at least 3 hours prior to the test. The volunteers also did not eat during the test. At the beginning of the test each person brushed his teeth with a popular benchmark toothpaste, 1 hour before the test.

DNA was collected with Zymo Research R1104/R1106 swabs with tubes. The swabs were applied for 30 seconds over the teeth, cheeks and tongue.

The subjects were informed about the importance and the meaning of the study. Written informed consent was obtained from all subjects prior to entry into the trial. Following criteria were used for subject selection: >= 18 years old and clinically healthy (no mouth diseases); For exclusion: oral diseases or pregnancy.

The conditions of the test were kept hygienic by sterilization of laboratory tools and glass and by boiling the water used for the mouthwash for 5 min prior to its use. Furthermore, a HEPA filter was used to limit the presence of foreign microorganisms in the air and the researcher wore gloves and mouth mask at all times.

The procedure was as follows:

### Time: 0h

1) The oral microbiome of each subject was sampled with a swab. The swab was rubbed well on gums, teeth and tong. The swab was then closed in a tube with buffer and placed immediately in a freezer at -18°C.
2) Immediately after the sampling each subject had to keep in his mouth 50% glucose solution for 30 seconds.
3) Immediately after this rinsing with glucose, each volunteer had to chew for 15 minutes a chewing gum.
4) The second sample was taken after 1 hour. The swab was rubbed well on gums, teeth and tong. The swab was then closed in a tube with buffer and placed immediately in a freezer at - 18°C.

The same procedure was repeated the next day for the chewing gum containing 2% prebiotic.

The swabs were analysed by genomic DNA isolation and bacteria 16s rRNA gene (V3-V4) PCR amplification and Illumina sequencing (Illumina MiSeq 2x300 bp).

The raw data includes Illumina sequenced reads presented as an abundance summary for all taxonomic paths and classification per unique pseudoread.

### Results

The disturbance of the total microbiome was evaluated by taking into account the percentage of species of the total microbiome that are out of balance (i.e. wherein the abundance has changed more than 30%) and is shown in Table 5.

**Table 5. Microbiome disturbance after glucose treatment**

| Disturbed microbiome for blank trial | Disturbed microbiome for trial with inulin |
|---|---|
| 85.75% | 57.88% |

When the oral microbiome is disturbed with glucose and a blank chewing gum is used, 1 hour later about 85% of the bacteria is still out of balance. For the same test, when using a chewing gum comprising 2% inulin as a prebiotic, after 1 hour, about 58% of the total microbiome is out of balance (Table 5).

The influence on the abundance of pathogens causing malodor is shown in Table 6. The following bacteria species were evaluated: *Prevotella melaninogenica, Fusobacterium nucleatum, Centipeda periodontii, Eikenella corrodens, Porphyromonas endodontalis, Tannerella forsythia.*

**Table 6. Influence on abundance of pathogens causing malodor**

| Blank | | 2% inulin | |
|---|---|---|---|
| 0 min | 60 min | 0 min | 60 min |
| 1.66% | 1.67% | 2.86% | 0.98% |

Comparison of abundance of the bacteria responsible for malodor shows that the presence of inulin tends to control the abundance of these pathogenic bacteria, resulting in about a 2/3 decrease in the undesirable bacteria.

In conclusion, the present experiment demonstrates the supporting, restoring and rebalancing power of a prebiotic in a daily oral care product or in a chewing gum after a disturbance, such as after using foodstuff/drinks associated with caries, gingivitis and other oral diseases/disorders.

## Claims

1. Prebiotic compound for use in a method for protecting, maintaining and/or restoring a healthy microbiota or a beneficial bacteria population in an oral cavity of a subject and/or for use in preventing or mitigating an oral cavity disorder,
wherein the prebiotic compound is administered to said oral cavity as an oral care composition, wherein the composition comprises between 0.1 and 20 wt% of the prebiotic compound, based on the total weight of the composition,
wherein said prebiotic compound is an oligo- or polysaccharide consisting of between 2 and 100 monosaccharide units, selected from the group consisting of fructan, galactan, kefiran, fructooligosaccharides, galactooligosaccharides, xylooligosaccharides, human milk oligosaccharides and isomaltooligosaccharides.

2. Prebiotic compound for use according to claim 1, wherein said prebiotic compound is an oligosaccharide consisting of between 2 and 20 monosaccharide units, preferably between 2 and 10 monosaccharide units.

3. Prebiotic compound for use according to claim 1 or 2, wherein the prebiotic compound is inulin or fructooligosaccharides.

4. Prebiotic compound for use according to any one of claim 1 to 3, wherein said method is a method for at least partially negating the effect of an oral microbiota disrupting compound on an oral bacterial population, wherein the oral bacterial population in said oral cavity is or has been contacted with said oral microbiota disrupting compound.

5. Prebiotic compound for use according to claim 4, wherein said microbiota disrupting compound is in contact with said oral cavity simultaneously with or prior to administration of the oral care composition.

6. Prebiotic compound for use according to any one of claims 1 to 5, wherein the oral care composition further comprises an oral microbiota disrupting compound, particularly wherein said oral microbiota disrupting compound is ethanol, an antimicrobial agent, such as triclosan, chlorhexidine or a quaternary ammonium compound, or a surfactant.

7. Prebiotic compound for use according to any one of claims 1 to 6, wherein the oral care composition comprises between 0.5 and 10 wt% of said prebiotic compound, based on the total weight of the composition.

8. Prebiotic compound for use according to any one of claims 1 to 7, wherein the oral care composition further comprises one or more additional ingredients selected from a fluoride ion source, remineralization agent, abrasives, diluents, pH modifying or buffering agents, surfactants, foaming agents, thickening agents, humectants, sweeteners, pigments, flavouring agents, fragrances, and preserving agents.

9. Prebiotic compound for use according to any one of claims 1 to 8, wherein the oral care composition is a mouth wash, a tooth paste or tooth gel, a mouth spray, a powder, a lozenge, an oral tablet, or chewing gum.

10. Prebiotic compound for use according to claim 9, wherein the composition is a mouthwash comprising between 0.1 and 20 wt% of the prebiotic compound, based on the total weight of the composition, wherein the prebiotic compound is selected from the group fructan, galactan, kefiran, fructooligosacchires, galactooligosaccharides, xylooligosaccharides, human milk oligosaccharides and isomaltooligosaccharides, preferably wherein the prebiotic compound is inulin or fructooligosaccharides, and further comprising an aqueous ethanol solution or an antimicrobial agent.

11. Prebiotic compound for use according to claim 9, wherein the composition is a toothpaste comprising between 0.1 and 20 wt% of the prebiotic compound, wherein the prebiotic compound is selected from the group fructan, galactan, kefiran, fructooligosaccharides, galactooligosaccharides, xylooligosaccharides, human milk oligosaccharides and isomaltooligosaccharides, preferably wherein the prebiotic compound is inulin or fructooligosaccharides, and further comprising an antimicrobial agent.

12. Prebiotic compound for use according to any one of claims 1 to 11, wherein the oral cavity disorder is caries, gingivitis, or periodontitis.

## Patentansprüche

1. Präbiotische Verbindung zur Verwendung bei einem Verfahren zum Schützen, Aufrechterhalten und/oder Wiederherstellen einer gesunden Mikrobiota oder einer nützlichen Bakterienpopulation in einer Mundhöhle eines Individuums und/oder zur Verwendung zum Verhindern oder Abschwächen einer Mundhöhlenstörung,
wobei die präbiotische Verbindung an die Mundhöhle als eine Mundpflegezusammensetzung verabreicht wird, wobei die Zusammensetzung zwischen 0,1 und 20 Gew.-% an der präbiotischen Verbindung, bezogen auf das Gesamtgewicht der Zusammensetzung, umfasst,
wobei die präbiotische Verbindung ein Oligo- oder Polysaccharid ist, das aus zwischen 2 und 100 Monosaccharideinheiten ausgewählt aus der Gruppe bestehend aus Fructan, Galactan, Kefiran, Fructooligosacchariden, Galactooligosacchariden, Xyloligosacchariden, menschlichen Milcholigosacchariden und Isomaltooligosacchariden besteht.

2. Präbiotische Verbindung zur Verwendung nach Anspruch 1, wobei die präbiotische Verbindung ein Oligosaccharid ist, das aus zwischen 2 und 20 Monosaccharideinheiten, vorzugsweise zwischen 2 und 10 Monosaccharideinheiten, besteht.

3. Präbiotische Verbindung zur Verwendung nach Anspruch 1 oder 2, wobei es sich bei der präbiotischen Verbindung um Inulin oder Fructooligosaccharide handelt.

4. Präbiotische Verbindung zur Verwendung nach einem der Ansprüche 1 bis 3, wobei das Verfahren ein Verfahren zum wenigstens teilweisen Aufheben der Wirkung einer Verbindung, die orale Mikrobiota stört, auf eine orale Bakterienpopulation ist, wobei die orale Bakterienpopulation in der Mundhöhle mit der Verbindung, die orale Mikrobiota stört, in Kontakt gebracht wird oder worden ist.

5. Präbiotische Verbindung zur Verwendung nach Anspruch 4, wobei die Mikrobiota-störende Verbindung gleichzeitig mit oder vor der Verabreichung der Mundpflegezusammensetzung mit der Mundhöhle Kontakt bildet.

6. Präbiotische Verbindung zur Verwendung nach einem der Ansprüche 1 bis 5, wobei die Mundpflegezusammensetzung ferner eine Verbindung, die orale Mikrobiota stört, umfasst, insbesondere wobei die Verbindung, die orale Mikrobiota stört, Ethanol, ein antimikrobielles Mittel, wie z.B. Triclosan, Chlorhexidin oder eine quartäre Ammoniumverbindung, oder ein Tensid ist.

7. Präbiotische Verbindung zur Verwendung nach einem der Ansprüche 1 bis 6, wobei die Mundpflegezusammensetzung zwischen 0,5 und 10 Gew.-% an der präbiotischen Verbindung, bezogen auf das Gesamtgewicht der Zusammensetzung, umfasst.

8. Präbiotische Verbindung zur Verwendung nach einem der Ansprüche 1 bis 7, wobei die Mundpflegezusammensetzung ferner einen oder mehrere zusätzliche Bestandteile ausgewählt aus einer Fluoridionenquelle, einem Remineralisierungsmittel, Abrasivstoffen, Verdünnungsmitteln, pH-modifizierenden oder puffernden Mitteln, Tensiden, Schaummitteln, Verdickungsmitteln, Feuchthaltemitteln, Süßungsmitteln, Pigmenten, Aromastoffen, Duftstoffen und Konservierungsmitteln umfasst.

9. Präbiotische Verbindung zur Verwendung nach einem der Ansprüche 1 bis 8, wobei die Mundpflegezusammensetzung eine Mundspülung, eine Zahnpaste oder ein Zahngel, ein Mundspray, ein Pulver, eine Lutschtablette, eine orale Tablette oder Kaugummi ist.

10. Präbiotische Verbindung zur Verwendung nach Anspruch 9, wobei die Zusammensetzung eine Mundspülung ist, die zwischen 0,1 und 20 Gew.-% an der präbiotischen Verbindung, bezogen auf das Gesamtgewicht der Zusammensetzung, umfasst, wobei die präbiotische Verbindung ausgewählt ist aus der Gruppe Fructan, Galactan, Kefiran, Fructooligosaccharide, Galactooligosaccharide, Xyloligosaccharide, menschliche Milcholigosaccharide und Isomaltooligosaccharide, wobei die präbiotische Verbindung vorzugsweise Inulin oder Fructooligosaccharide ist, und ferner umfassend eine wässrige Ethanollösung oder ein antimikrobielles Mittel.

11. Präbiotische Verbindung zur Verwendung nach Anspruch 9, wobei die Zusammensetzung eine Zahnpasta ist, die zwischen 0,1 und 20 Gew.-% an der präbiotischen Verbindung umfasst, wobei die präbiotische Verbindung ausgewählt ist aus der Gruppe Fructan, Galactan, Kefiran, Fructooligosaccharide, Galactooligosaccharide, Xyloligosaccharide, menschliche Milcholigosaccharide und Isomaltooligosaccharide, wobei die präbiotische Verbindung vorzugsweise Inulin oder Fructooligosaccharide ist, und ferner umfassend ein antimikrobielles Mittel.

12. Präbiotische Verbindung zur Verwendung nach einem der Ansprüche 1 bis 11, wobei die Mundhöhlenstörung Karies, Gingivitis oder Parodontitis ist.

## Revendications

1. Composé prébiotique pour utilisation dans un procédé de protection, maintien et/ou restauration d'un microbiote sain ou d'une population de bactéries bénéfiques dans la cavité buccale d'un sujet et/ou pour utilisation pour prévenir ou atténuer un trouble de la cavité buccale,
dans lequel le composé prébiotique est administré à ladite cavité buccale en tant que composition de soins buccaux, la composition comprenant entre 0,1 et 20 % en poids du composé prébiotique sur la base du poids total de la composition,
dans lequel ledit composé prébiotique est un oligo- ou polysaccharide constitué d'entre 2 et 100 unités de monosaccharide, choisi dans le groupe constitué par le fructane, le galactane, le kéfirane, les fructooligosaccharides, les galactooligosaccharides, les xylooligosaccharides, les oligosaccharides du lait humain et les isomaltooligosaccharides.

2. Composé prébiotique pour utilisation selon la revendication 1, dans lequel ledit composé prébiotique est un oligosaccharide constitué d'entre 2 et 20 unités de monosaccharide, de préférence entre 2 et 10 unités de monosaccharide.

3. Composé prébiotique pour utilisation selon la revendication 1 ou 2, dans lequel le composé prébiotique est l'inuline ou des fructooligosaccharides.

4. Composé prébiotique pour utilisation selon l'une quelconque des revendications 1 à 3, dans lequel ledit procédé est un procédé pour neutraliser au moins partiellement l'effet d'un composé perturbant le microbiote buccal sur une population bactérienne buccale, dans lequel la population bactérienne buccale dans ladite cavité buccale est ou a été mise en contact avec ledit composé perturbant le microbiote buccal.

5. Composé prébiotique pour utilisation selon la revendication 4, dans lequel ledit composé perturbant le microbiote est en contact avec ladite cavité buccale simultanément avec ou avant l'administration de la composition de soins buccaux.

6. Composé prébiotique pour utilisation selon l'une quelconque des revendications 1 à 5, dans lequel la composition de soins buccaux comprend en outre un composé perturbant le microbiote buccal, en particulier dans lequel ledit composé perturbant le microbiote buccal est l'éthanol, un agent antimicrobien, tel que le triclosan, la chlorhexidine ou un composé d'ammonium quaternaire, ou un tensioactif.

7. Composé prébiotique pour utilisation selon l'une quelconque des revendications 1 à 6, dans lequel la composition de soins buccaux comprend entre 0,5 et 10 % en poids dudit composé prébiotique, par rapport au poids total de la composition.

8. Composé prébiotique pour utilisation selon l'une quelconque des revendications 1 à 7, dans lequel la composition de soins buccaux comprend en outre un ou plusieurs ingrédients supplémentaires choisis parmi une source d'ions fluorure, un agent de reminéralisation, des abrasifs, des diluants, des agents modifiant le pH ou tampons, des tensioactifs, des agents moussants, des agents épaississants, des humectants, des édulcorants, des pigments, des agents aromatisants, des fragrances et des conservateurs.

9. Composé prébiotique pour utilisation selon l'une quelconque des revendications 1 à 8, dans lequel la composition de soins buccaux est un bain de bouche, une pâte dentifrice ou un gel dentaire, un spray buccal, une poudre, une pastille, un comprimé oral, ou un chewing gum.

10. Composé prébiotique pour utilisation selon la revendication 9, dans lequel la composition est un bain de bouche comprenant entre 0,1 et 20 % en poids du composé prébiotique, sur la base du poids total de la composition, dans lequel le composé prébiotique est choisi dans le groupe fructane, galactane, kéfirane, fructooligosaccharides, galactooligosaccharides, xylooligosaccharides, oligosaccharides du lait humain et isomaltooligosaccharides, de préférence dans lequel le composé prébiotique est l'inuline ou des fructooligosaccharides, et comprenant en outre une solution aqueuse d'éthanol ou un agent antimicrobien.

11. Composé prébiotique pour utilisation selon la revendication 9, dans lequel la composition est une pâte dentifrice comprenant entre 0,1 et 20 % en poids du composé prébiotique, dans lequel le composé prébiotique est choisi dans le groupe fructane, galactane, kéfirane, fructooligosaccharides, galactooligosaccharides, xylooligosaccharides, oligosaccharides du lait humain et isomaltooligosaccharides, de préférence dans lequel le composé prébiotique est l'inuline ou des fructooligosaccharides, et comprenant en outre un agent antimicrobien.

12. Composé prébiotique pour utilisation selon l'une quelconque des revendications 1 à 11, dans lequel le trouble de la cavité buccale est une carie, une gingivite ou une parodontite.
